(19) Europäisches Patentamt
European Patent Office
Office européen des brevets

(11) **EP 4 410 323 A1**

(12) **DEMANDE DE BREVET EUROPEEN**

(43) Date de publication:
**07.08.2024 Bulletin 2024/32**

(21) Numéro de dépôt: **24155948.3**

(22) Date de dépôt: **06.02.2024**

(51) Classification Internationale des Brevets (IPC):
**A61L 27/36** (2006.01) **A61L 27/50** (2006.01)

(52) Classification Coopérative des Brevets (CPC):
**A61L 27/3604; A61L 27/3645; A61L 27/3662; A61L 27/3679; A61L 27/50;** A61L 2430/06; A61L 2430/10; A61L 2430/22; A61L 2430/30; A61L 2430/34

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **06.02.2023 FR 2301113**
**15.01.2024 FR 2400351**

(71) Demandeur: **TBF Genie Tissulaire (TBF)**
**69780 Mions (FR)**

(72) Inventeurs:
• **BARNOUIN, Laurence**
**38460 VENERIEU (FR)**
• **GROSSETETE, Florine**
**69003 Lyon (FR)**

(74) Mandataire: **Tripoz, Inès**
**Cabinet Tripoz**
**Le Pôle Sud**
**22 rue Seguin**
**69002 Lyon (FR)**

(54) **STRUCTURE TRESSÉE COMPRENANT DES ÉLÉMENTS DE TYPE FILAIRE CONSTITUÉS D'AU MOINS UNE COUCHE MEMBRANAIRE ISSUE DE TISSUS PLACENTAIRES**

(57) La présente invention concerne des structures tressées comprenant des éléments de type filaire constitués d'au moins une couche membranaire issue de tissus placentaires ladite structure tissée ayant un taux d'humidité supérieur ou égal à 13%, pouvant être en particulier obtenues par un procédé comprenant au moins étape de séchage par dessication.

La présente invention concerne également l'utilisation en thérapeutique ou en chirurgie ainsi que le procédé d'obtention de telles structures.

FIGURE 5

EP 4 410 323 A1

**Description**

La présente invention concerne le domaine de l'ingénierie tissulaire, plus particulièrement elle concerne un procédé de préparation de structures tressées comprenant des éléments de type filaire constitués d'au moins une couche membranaire issue de tissus placentaires de mammifère, ces structures tressées étant adaptées pour servir de greffon en chirurgie.

La membrane chorioamniotique, qui sépare le foetus de l'endomètre de la mère chez les mammifères, inclut la membrane amniotique, ou amnios, et la membrane choriale, ou chorion. Ces deux membranes sont reliées par une membrane tissulaire spongieuse, aussi appelée couche spongieuse, constituée entre autres de collagène et de protéoglycanes, la membrane tissulaire spongieuse comportant des ponts protéiques, attachés de part et d'autre à l'amnios d'une part, et au chorion, d'autre part.

La membrane amniotique est la couche la plus interne de la membrane chorioamniotique. Elle a pour rôle de protéger le foetus et de maintenir autour de lui le liquide amniotique. Cette membrane amniotique est un tissu très fin, transparent qui comporte plusieurs couches, à savoir une couche épithéliale, une membrane basale, une couche compacte et une couche fibroblastique.

La membrane choriale est la couche la plus externe de la membrane chorioamniotique, celle qui se trouve immédiatement en contact avec la paroi de l'utérus maternel (le decidua). Cette membrane choriale est un tissu épais et fibreux, comportant également plusieurs couches, à savoir une couche réticulaire, une membrane basale et une couche trophoblastique.

La membrane amniotique, obtenue à partir du placenta après un accouchement, est un tissu utilisé depuis plus de cent ans dans le traitement des brûlures et des blessures. En effet, dès 1910, Davies utilisait des membranes foetales tant sur des brûlures que sur des tissus ulcérés. Trelford et Trelford-Sauder rapportent qu'en 1935 des auteurs ont publié des applications cliniques de la membrane amniotique en vaginoplastie, reconstruction conjonctivale, traitement des brûlures ou des blessures, et traitements relatifs à l'adhérence intra-abdominale. C'est en 1940 que De Roetth a utilisé pour la première fois une membrane foetale en ophtalmologie reconstructive pour traiter des altérations conjonctivales de patients. Trelford *et al.* rapportent aussi qu'en 1952 Douglas a utilisé de l'amnios pour traiter des blessures étendues. Pour la première fois, il fut indiqué que la couche stromale de la membrane amniotique, comprenant la couche compacte et la couche fibroblastique, permettait une adhérence plus importante de la greffe, et donc de son efficacité. En 1972, les travaux de Trelford *et al.* ont confirmé ce fait. Gindraux *et al.* rapportent qu'à partir de 1972, et surtout depuis sa redécouverte en 1995, d'autres auteurs ont confirmé toutes les applications cliniques présentées précédemment, et ont également signalé de nouvelles indications telles que le tractus génito-urinaire, l'estomac, le larynx, la cavité orale, la tête et le cou, que ce soit dans des essais cliniques ou des rapports de cas.

Non vascularisée et non innervée, la membrane amniotique est riche en collagène et en divers facteurs de croissance, et présente des propriétés participant au processus de cicatrisation.

La demande de brevet WO2017/140914 déposée au nom de la demanderesse divulgue notamment un procédé de préparation d'un matériau d'allogreffe obtenu à partir de tissus placentaires issus de mammifère, ledit procédé comprenant la lyophilisation desdits tissus placentaires. La formation de structure tressée n'est jamais abordée dans cette demande.

Les techniques de génie tissulaire sont notamment employées pour la formation de bandelettes sous-urétrales.

Différents types de matériaux synthétiques et organiques sont classiquement utilisés pour la fabrication de bandelettes sous-urétrales. Leurs avantages et inconvénients sont divulgués dans la publication Deval et al., J Gynecol Obstet Biol Reprod 2002 ; 31 : 131-143.

Concernant les matériaux biologiques, des auto, allo et xéno greffes ont été utilisées, par exemple les greffes d'aponévrose antérieure des muscles droits de l'abdomen, de fascia lata, de derme, de derme de porc, de dure-mère. Globalement l'utilisation de ces matériaux biologiques ne s'est pas révélée efficace du fait d'un fort taux d'échec et de récidives pour certains, en cause notamment une élasticité moyenne voire une rigidité de ces greffes.

C'est pourquoi les bandelettes sous-urétrales synthétiques sont le plus largement utilisées. Le matériau synthétique le plus couramment utilisé est du polypropylène tricoté monofilament ou multifilament car il induit peu de rejets et d'intolérances. Différents types de bandelettes synthétiques sont décrites dans la littérature. Néanmoins ces bandelettes synthétiques ne sont pas exemptes pour autant d'inconvénients et des complications après pose peuvent survenir, notamment en raison du gain en élasticité des matériaux synthétiques, ce gain étant lié aux mèches monofilaments.

La demande de brevet WO2021234138 déposée au nom de la demanderesse, divulgue notamment des bandelettes sous-urétrales obtenues à partir d'un vaisseau ombilical ou d'un segment de celui-ci. Elle ne divulgue pas l'utilisation de couches membranaires issues de tissus placentaires ni de structures tressées.

Bien qu'efficace, ces bandelettes obtenues à partir d'un vaisseau ombilical présentent l'inconvénient d'être légèrement trop fragiles et en particulier trop élastiques pour entourer et fermer efficacement le col vésical. Ces inconvénients sont exacerbés dans le cas de leurs utilisations en tant que matériaux d'allogreffe dans le domaine orthopédique, notamment dans les greffes ligamentaires ou tendineuses, mais également en tant que renfort des structures pelviennes.

De manière tout à fait inattendue, la demanderesse a mis au point une structure tressée comprenant des éléments de type filaire constitués d'au moins une couche membranaire issue de tissus placentaires permettant de palier à ces inconvénients.

La production par génie tissulaire de structures tressées est connue pour des applications différentes, en particulier pour la formation de structures vasculaires.

La demande de brevet WO2008/061261 A2 divulgue la production de fils biologiques synthétisées à partir de feuillets de matrices extracellulaire obtenus par culture cellulaire, en particulier par des fibroblastes, et notamment leurs utilisations en tant que fils de suture ainsi que leur tressage.

La publication Grémare A, et al, Biofabrication, 22 aout 2022 ; 14(4) divulgue la fabrication fils constitués de membrane amniotique. Dans ce procédé de fabrication, les membranes amniotiques sont séchées selon un procédé classique de séchage à température ambiante. Les fils obtenus sont tissés pour la fabrication de greffons vasculaires. Les structures obtenues sont donc logiquement imperméables et peu déformables de façon à pouvoir contenir l'afflux sanguin.

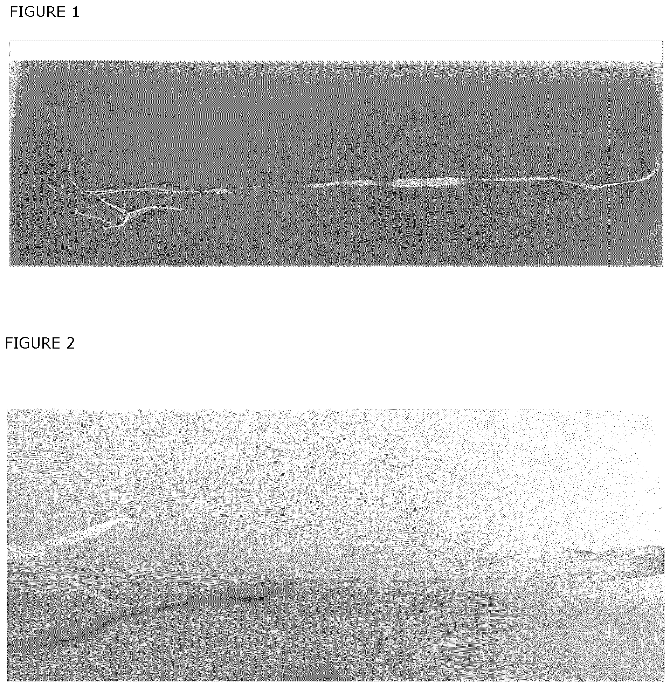

Cependant, les produits obtenus selon le procédé décrit dans cette dernière publication, c'est-à-dire par séchage à température ambiante, ne permettent pas d'obtenir un matériau adapté pour leur utilisation dans les domaines suscités, notamment pour le renfort du sphincter urinaire dans les pathologies d'incontinence neurologique. En effet, le maillage serré employé pour la formation de vaisseaux entraine une faible déformabilité, et donc une grande rigidité opposée à la flexibilité souhaitée. Ainsi, lors du tressage en maillage plus lâche, celui-ci devient inconstant et la structure conserve sa rigidité, est donc peu élastique et difficile à manipuler comme illustré en Figure 1 et Figure 2.

De manière tout à fait surprenante, la demanderesse a mis au point une technique qui, en faisant varier la teneur en eau de la structure tressée, résout l'ensemble des problèmes connus de l'art antérieur. La demanderesse a par ailleurs mis au point un procédé qui se démarque de l'art antérieur en ce qu'il comprend une étape de séchage par dessication permettant de résoudre l'ensemble des problèmes techniques de l'art antérieur.

La structure tressée ainsi obtenue présente à la fois une épaisseur et une souplesse supérieure permettant notamment l'absence de marquage lors du pliage, tout en conservant une résistance adaptée pour son utilisation en chirurgie, notamment en tant que greffon en urologie ou en orthopédie.

Enfin, la structure selon la présente invention pourra également être utilisé en tant qu'implant de renfort du plancher pelvien, en particulier dans le cadre du traitement des prolapsus des organes pelviens. Pour de telles applications, en raison de sa souplesse, la structure tressée pourra être implanté chirurgicalement que ce soit par voie péritonéale ou abdominale (voie haute) mais également par voie basse.

Les Figure 1 et Figure 2 illustrent des structures tressées à l'aide d'un métier à tisser comprenant des éléments de type filaire constitués d'au moins une couche membranaire issue de tissus placentaires obtenue par séchage à l'air libre, sans étape de séchage par dessication. Ces images démontrent l'aspect irrégulier, plus difficilement manipulable et implantable de la structure tressée obtenue avec une étape de séchage à l'air libre.

La Figure 3 illustre une comparaison une couche membranaire couche membranaire issue de tissus placentaires séchée par dessication (image A), par lyophilisation (image B), ou par séchage à l'air libre (image C).

La Figure 4 illustre une structure tressée sur un métier à tisser obtenue selon le procédé selon l'invention.

La Figure 5 illustre une structure tressée obtenue selon le procédé selon l'invention.

La Figure 6 illustre une couche membranaire issue de tissus placentaires séchée par dessication et pliée sur elle-même le long de sa diagonale.

La Figure 7 illustre une couche membranaire issue de tissus placentaires séchée par lyophilisation et pliée sur elle-même le long de sa diagonale.

La présente invention concerne un procédé de préparation d'une structure tressée comprenant des éléments de type filaire constitués d'au moins une couche membranaire issue de tissus placentaires comprenant les étapes de :

a) On dispose d'au moins une couche membranaire issue de tissus placentaires,
b) On découpe la ou les couche(s) membranaire(s) issue(s) de tissus placentaires,
c) On obtient des éléments de type filaire constitués d'au moins une couche membranaire issue de tissus placentaires,
d) On tresse les éléments de type filaire,
e) On obtient une structure tressée comprenant des éléments de type filaire constitués d'au moins une couche membranaire issue de tissus placentaires, ledit procédé comprenant au moins une étape de séchage par dessication de la ou des couche(s) membranaire(s) issue(s) de tissus placentaires après l'étape a) et/ou des éléments de type filaire obtenus à l'étape c) et/ou de la structure tressée après l'étape e).

Dans un mode de réalisation, le procédé de préparation d'une structure tressée comprenant des éléments de type filaire constitués d'au moins une couche membranaire issue de tissus placentaires selon l'invention comprend les étapes de :

a) On dispose d'au moins une couche membranaire issue de tissus placentaires,

b) On sèche la ou les couche(s) membranaire(s) issue(s) de tissus placentaires de l'étape a) par au moins une étape de séchage par dessication,

c) On découpe la ou les couche(s) membranaires issue(s) de tissus placentaires en éléments de type filaire,

d) On obtient des éléments de type filaire constitués d'au moins une couche membranaire issue de tissus placentaires,

e) On tresse les éléments de type filaire,

f) On obtient une structure tressée comprenant des éléments de type filaire constitués d'au moins une couche membranaire issue de tissus placentaires.

Dans un mode de réalisation, le procédé de préparation d'une structure tressée comprenant des éléments de type filaire constitués d'au moins une couche membranaire issue de tissus placentaires selon l'invention comprend les étapes de :

a) On dispose d'au moins une couche membranaire issue de tissus placentaires,

b) On découpe la ou les couche(s) membranaire(s) issue(s) de tissus placentaires,

c) On obtient des éléments de type filaire constitués d'au moins une couche membranaire issue de tissus placentaires,

d) On sèche les éléments de type filaire obtenus à l'étape de l'étape c) par au moins une étape de séchage par dessication,

e) On tresse les éléments de type filaire,

f) On obtient une structure tressée comprenant des éléments de type filaire constitués d'au moins une couche membranaire issue de tissus placentaires.

Dans un mode de réalisation, le procédé de préparation d'une structure tressée comprenant des éléments de type filaire constitués d'au moins une couche membranaire issue de tissus placentaires selon l'invention comprend les étapes de :

a) On dispose d'au moins une couche membranaire issue de tissus placentaires,

b) On découpe la ou les couche(s) membranaire(s) issue(s) de tissus placentaires

c) On obtient des éléments de type filaire constitués d'au moins une couche membranaire issue de tissus placentaires,

d) On tresse les éléments de type filaire,

e) On obtient une structure tressée comprenant des éléments de type filaire constitués d'au moins une couche membranaire issue de tissus placentaires.

f) On sèche la structure tressée comprenant des éléments de type filaire constitués d'au moins une couche membranaire issue de tissus placentaires obtenue à l'étape e) par au moins une étape de séchage par dessication.

Ainsi, dans un mode de réalisation, l'étape de séchage par dessication est réalisée sur la structure tressée comprenant des éléments de type filaire constitués d'au moins une couche membranaire issue de tissus placentaires obtenue à l'étape e).

Au sens de la présente invention, l'expression « au moins un(e) » signifie « un(e) ou plusieurs » et pourra être remplacée par cette dernière sans distinction.

Au sens de la présente invention, on entend par « couche membranaire issue de tissus placentaires » l'un des feuillets tissulaires constitutifs du placenta.

Dans un mode de réalisation, au moins une couche membranaire issue de tissus placentaires comprend de la membrane amniotique, de la membrane choriale, de la membrane tissulaire spongieuse et/ou de la membrane chorioamniotique, chacun de ces feuillets pouvant inclure, ou non, les sous-structures physiologiques constitutives de l'amnios et/ou du chorion.

Dans un mode de réalisation, au moins une couche membranaire issue de tissus placentaires dont on dispose à l'étape a) est d'origine animale ou humaine.

Dans un mode de réalisation, au moins une couche membranaire issue de tissus placentaires dont on dispose à l'étape a) est d'origine humaine.

Dans un mode de réalisation, au moins une couche membranaire issue de tissus placentaires dont on dispose à l'étape a) comprend tout ou partie de la membrane amniotique et/ou tout ou partie de la membrane choriale.

Dans un mode de réalisation, la couche membranaire issue de tissus placentaires dont on dispose à l'étape a) est une membrane amniotique humaine.

Au sens de la présente invention, on entend par « membrane amniotique », ou « amnios » l'enveloppe tissulaire qui se développe autour de l'embryon, puis du foetus, chez le mammifère durant la grossesse. Elle a pour rôle de protéger l'organisme en développement en maintenant autour de lui le liquide amniotique. Elle est accolée à la deuxième membrane qui est le chorion.

Dans un mode de réalisation, au moins une couche membranaire issue de tissus placentaires dont on dispose à l'étape a) comprend au moins une des sous-couches physiologiques de la membrane amniotique choisies dans le groupe comprenant la couche cellulaire épithéliale, la membrane basale, la couche compacte, la couche fibroblastique et/ou la couche spongieuse.

Dans un mode de réalisation préférentiel, au moins une couche membranaire issue de tissus placentaires dont on dispose à l'étape a) comprend de la membrane amniotique comprenant sa couche spongieuse.

Dans un mode de réalisation, au moins une couche membranaire issue de tissus placentaires dont on dispose à l'étape a) est constituée de la membrane amniotique comprenant sa couche spongieuse.

Au sens de la présente invention, on entend par « membrane choriale », ou « chorion » l'enveloppe tissulaire située entre l'amnios et la paroi utérine qui se développe autour de l'embryon, puis du foetus, chez le mammifère durant la grossesse.

Dans un mode de réalisation, au moins une couche membranaire issue de tissus placentaires dont on dispose à l'étape a) comprend au moins une les sous-couches physiologiques de la membrane choriale choisi dans le groupe comprenant la couche réticulaire, la membrane basale et/ou la couche cellulaire trophoblastique.

Au sens de la présente invention, on entend par « membrane chorioamniotique » l'ensemble amnios et chorion, qui n'ont pas été séparés naturellement ou artificiellement, ceux-ci étant reliés par des ponts protéiques.

Au sens de la présente invention, on entend par « membrane tissulaire spongieuse », ou « couche spongieuse » une couche située entre l'amnios et le chorion.

Dans un mode de réalisation, la couche membranaire issue de tissus placentaires dont on dispose à l'étape a) est préparée selon le protocole tel que décrit dans la demande WO2017140914A1.

La couche membranaire issue de tissus placentaires selon la présente invention est obtenue après l'accouchement d'un donneur proprement informé et consentant en accord avec exigences des directives Européennes et la Loi Bioéthique. Cet accouchement peut s'être déroulé par césarienne ou par voie basse.

Par exigence sanitaire relative aux dons de tissus et de cellules d'origine humaine, une qualification socioclinique du donneur et biologique en amont du donneur est systématique. Cette qualification implique une recherche de virus HIV, hépatites B, C, HTLV et de la bactérie tréponème pâle responsable de la syphilis.

Dans un mode de réalisation, l'un quelconque des procédés selon l'invention comprend une étape préalable de séparation de la ou des couche(s) membranaire(s) issue(s) de tissus placentaires.

Dans un mode de réalisation, au moins une couche membranaire issue de tissus placentaires dont on dispose à l'étape a) est préalablement congelée.

Dans un mode de réalisation, le tissu placentaire est préalablement placé dans une boîte stérile, congelé et transporté à une température de -20°C, puis stocké à -80°C.

Dans un mode de réalisation, le tissu placentaire est transporté à +4 °C.

Dans un mode de réalisation, le tissu placentaire n'a pas été congelé initialement, la découpe et la séparation de la ou des couche(s) membranaire(s) issue(s) de tissus placentaires sont réalisées après le transport à +4°C.

Dans un mode de réalisation, au moins une couche membranaire issue de tissus placentaires est conservée à -80°C.

Dans un mode de réalisation, le placenta non séparé de ses tissus physiologiques peut être congelé pour conservation à une température de -80°C pendant une durée allant jusqu'à 2 ans.

Dans un mode de réalisation, au moins une couche membranaire issue de tissus placentaires à l'état congelé est ensuite lavée dans de l'eau purifiée ou séjourne dans un bain d'eau purifiée.

Cette étape assure tant la décongélation de la ou des couche(s) membranaire(s) issue(s) de tissus placentaires si nécessaire jusqu'à la température ambiante, qu'une lyse des cellules présentes dans le la ou les couche(s) membranaire(s) issue(s) de tissus placentaires par effet de pression osmotique.

Dans un mode de réalisation, au moins une couche membranaire issue de tissus placentaires dont on dispose à l'étape a) est viro-inactivée.

Dans un mode de réalisation, l'un quelconque des procédés selon l'invention comprend une étape a') de viro-inactivation de la ou des couche(s) membranaire(s) issue(s) de tissus placentaires suite à l'étape a).

Au sens de la présente invention, on entend par « viro-inactivation » une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN. Cette étape permet notamment de disposer d'un produit exempt d'agents microbiologiques tels que bactéries, virus, parasites.

Dans un mode de réalisation, l'étape a') de viro inactivation comprend une étape i) de viro-inactivation comprenant l'application sur la ou les couche(s) membranaire(s) issue(s) de tissus placentaires d'un lavage et/ou le séjour de cette dernière dans un bain, composé d'un premier agent viro-inactivant.

Dans un mode de réalisation, le premier agent viro-inactivant est l'éthanol.

Dans un mode de réalisation, le premier agent viro-inactivant est l'éthanol à une teneur en alcool de 50% à 80% (v/v) (50%≤ teneur en alcool ≤80%).

Dans un mode de réalisation, le premier agent viro-inactivant est l'éthanol à une teneur en alcool de 70% (v/v).

Dans un mode de réalisation, l'étape i) de viro-inactivation est effectuée pendant une durée comprise dans un intervalle allant de 30 minutes à 120 minutes (30 min≤ durée ≤120 min).

Dans un mode de réalisation, l'étape i) de viro-inactivation est effectuée pendant une durée comprise dans un intervalle allant de 45 minutes à 100 minutes (45 min≤ durée ≤100 min).

Dans un mode de réalisation, l'étape i) de viro-inactivation est effectuée pendant une durée d'environ 60 minutes.

Dans un mode de réalisation, l'étape i) de viro-inactivation est effectuée en traitant la ou les couche(s) membranaire(s) issue(s) de tissus placentaires avec de l'éthanol à une teneur en alcool de 70% (v/v) pendant une durée d'environ 60 minutes.

Dans un mode de réalisation, l'étape i) est suivi d'une étape i') de lavage de la couche membranaire issue de tissus placentaires par de l'eau purifiée ou un séjour dans un bain d'eau purifiée.

Dans un mode de réalisation, l'étape a') de viro inactivation comprend une seconde étape ii) de viro inactivation consécutive à l'étape i) ou i') comprenant l'application à la ou les couche(s) membranaire(s) issue(s) de tissus placentaires d'un lavage et/ou le séjour de cette dernière dans un bain, composé d'un deuxième agent de viro-inactivation.

Dans un mode de réalisation, le deuxième agent de viro inactivation est le peroxyde d'hydrogène.

Dans un mode de réalisation, le deuxième agent de viro-inactivation est le peroxyde d'hydrogène sous une forme choisie parmi une solution aqueuse, et un gaz.

Dans un mode de réalisation, le deuxième agent de viro-inactivation est le peroxyde d'hydrogène sous forme de solution aqueuse dans une concentration comprise dans un intervalle allant de 1% à 30% (w/v) (1% ≤ concentration w/v ≤ 30%).

Dans un mode de réalisation, le deuxième agent de viro-inactivation est le peroxyde d'hydrogène sous forme de solution aqueuse dans une concentration comprise dans un intervalle allant de 3% à 10% (w/v) (3% ≤ concentration w/v ≤ 10%).

Dans un mode de réalisation, l'étape ii) de viro-inactivation est effectuée pendant une durée comprise dans un intervalle allant de 30 minutes à 120 minutes (30 mn≤ durée ≤120 mn).

Dans un mode de réalisation, l'étape ii) de viro-inactivation est effectuée pendant une durée comprise dans un intervalle allant de 45 à 100 minutes (45 mn≤ durée ≤100 mn).

Dans un mode de réalisation, l'étape ii) de viro-inactivation est effectuée pendant une durée d'environ 60 minutes.

Dans un mode de réalisation, le procédé est caractérisé en ce que la deuxième étape de viro-inactivation ii) de la ou des couche(s) membranaire(s) issue(s) de tissus placentaires comprend deux sous-étapes de traitement par le deuxième agent de viro-inactivation choisi dans la famille des peroxydes.

Au sens de la présente invention, on entend par « peroxydes » tout composé chimique contenant un groupe fonctionnel de formule générale R-O-O-R' (deux atomes d'oxygène voisins liés entre eux) où R et R' sont des atomes d'hydrogène ou des radicaux alkyls quelconques.

Selon un mode de réalisation, la deuxième étape de viro-inactivation ii) de la ou des couche(s) membranaire(s) issue(s) de tissus placentaires comporte deux sous-étapes de traitement par le peroxyde d'hydrogène :

- une première sous-étape de traitement ii') s'effectuant avec une solution de peroxyde d'hydrogène à une concentration supérieure à 10% w/v pendant une durée d'au moins de 20 minutes ; et
- une deuxième sous-étape de traitement ii") s'effectuant avec une solution de peroxyde d'hydrogène à une concentration inférieure à 5 % w/v pendant une durée supérieure à 50 minutes.

Selon un autre mode de réalisation, la deuxième étape de viro-inactivation ii) de la ou des couche(s) membranaire(s) issue(s) de tissus placentaires comporte deux sous-étapes de traitement par le peroxyde d'hydrogène :

- une première sous-étape de traitement ii') s'effectuant avec une solution de peroxyde d'hydrogène à à une concentration de 30% w/v pendant une durée d'environ 15 minutes ; et
- une deuxième sous-étape de traitement ii')' s'effectuant avec une solution de peroxyde d'hydrogène à à une concentration de 3% w/v pendant une durée d'environ 60 minutes.

Dans un mode de réalisation, l'étape a') de viro inactivation comprend une troisième étape iii) consécutive à l'étape ii) comprenant au moins une étape de neutralisation, par application à la ou les couche(s) membranaire(s) issue(s) de tissus placentaires d'un lavage et/ ou d'un séjour de cette dernière dans un bain, composé d'au moins un tampon basique.

Dans un mode de réalisation, la troisième étape iii) comprend deux bains effectués dans un tampon basique contenant de l'hydroxyde de sodium dilué et dont le pH est ajusté à une valeur comprise dans un intervalle allant de 7 à 10 (7≤pH ≤10).

Dans un mode de réalisation, la troisième étape iii) comprend deux bains effectués dans un tampon basique contenant de l'hydroxyde de sodium dilué et dont le pH est ajusté à une valeur comprise dans un intervalle allant de 8 à 9 (8≤pH ≤9).

Dans un mode de réalisation, la troisième étape iii) comprend deux bains effectués dans un tampon basique contenant de l'hydroxyde de sodium dilué et dont le pH est ajusté à une valeur 8,5.

Dans un mode de réalisation, l'étape a') de viro inactivation comprend une dernière étape iv) comprenant l'application à la ou les couche(s) membranaire(s) issue(s) de tissus placentaires d'un lavage et/ou d'un séjour dans un bain, composé d'au moins une solution tampon assurant un rééquilibrage physiologique.

Dans un mode de réalisation, la dernière étape iv) comprend deux bains effectués en solution de PBS.

Dans un mode de réalisation, les étapes de décongélation, viro-inactivation, lavage, ajustage de pH et mise en tampon s'effectuent à température ambiante.

Dans un mode de réalisation, l'un quelconque des procédés selon l'invention comprend en outre une dernière étape de stérilisation de la structure tressée comprenant des éléments de type filaire constitués d'au moins une couche membranaire issue de tissus placentaires obtenue.

La stérilisation pourra être réalisé par toute méthode classiquement connue de l'Homme du métier.

Dans un mode de réalisation, la stérilisation est réalisée par irradiation.

Dans un mode de réalisation, la stérilisation est réalisée par irradiation par des rayonnement gamma.

Dans un mode de réalisation, la stérilisation est réalisée par irradiation par des rayonnements gamma à une dose comprise dans un intervalle allant de 25 à 35 kGrays (25 kGrays ≤ dose ≤ 35 kGrays).

Dans un mode de réalisation, la ou les couche(s) membranaire(s) issue(s) de tissus placentaires sont stériles.

Selon la présente invention, on entend par « stérile » une structure exempte de germe, à l'état naturel ou après stérilisation.

La stérilisation pourra être effectuée par toute les méthodes classiquement utilisées par l'Homme du métier.

Dans un mode de réalisation, la stérilisation est réalisée dans les conditions ci-dessus décrites.

Au sens de la présente invention, on entend par « dessication » l'élimination de l'eau contenue dans une substance, à l'aide de la chaleur, du vide et/ou d'une matière hygroscopique.

Au sens de la présente invention, on entend par « température positive » une température supérieure ou égale à 0°C (0°C ≤ température). En fonction de la pression les plages de température utilisées sont les plages de température ne permettant pas la formation de cristaux d'eau.

Ainsi, les dessiccations sous vide en températures négatives, telle que la cryodessiccation plus communément appelée lyophilisation, sont exclues de la définition de la dessication selon la présente invention.

Au sens de la présente invention, on entend par « lyophilisation » une technique visant à dessécher par sublimation un produit préalablement surgelé. Plus précisément, le liquide à ôter du produit est dans un premier temps transformé en solide (glace) par congélation ; puis par une dessiccation primaire, sous vide, le solide est sublimé ; enfin par une dessiccation secondaire, les molécules d'eau à la surface du produit sont extraites par désorption.

Dans un mode de réalisation, l'étape de séchage par dessication comprend l'application d'une part de la chaleur et d'autre part de vide et/ou d'une matière hygroscopique.

Dans un mode de réalisation, l'étape de séchage par dessication comprend l'application de chaleur et de vide.

Dans un mode de réalisation, l'étape de séchage par dessication ne comprend pas de congélation et/ou de surgélation de la structure placentaire et/ou ombilicale.

Dans un mode de réalisation, dans l'étape de séchage par dessication, la température minimale est choisie de manière à éviter la formation de cristaux d'eau en fonction de la pression appliquée.

Dans un mode de réalisation, l'étape de séchage par dessication est réalisée par montée progressive de la température à partir d'au moins 0°C (0°C ≤ température).

Dans un mode de réalisation, l'étape de séchage par dessication est réalisée par montée progressive de la température à partir d'au moins 2°C (2°C ≤ température).

Dans un mode de réalisation, l'étape de séchage par dessication est réalisée par montée progressive de la température à partir d'au moins 4°C (4°C ≤ température).

Dans un mode de réalisation, l'étape de séchage par dessication est réalisée par montée progressive de la température jusqu'à 35°C.

Dans un mode de réalisation, l'étape de séchage par dessication est réalisée par montée progressive de la température d'au moins 0°C à 35°C (0°C ≤ température ≤ 35°C).

Dans un mode de réalisation, l'étape de séchage par dessication est réalisée par montée progressive de la température dans un intervalle allant de 4°C à 35°C (4°C ≤ température ≤ 35°C).

Dans un mode de réalisation, l'étape de séchage par dessication est réalisée par montée progressive de la température par paliers de 1 à 10°C (1°C ≤ température ≤ 10°C).

Dans un mode de réalisation, l'étape de séchage par dessication est réalisée par montée progressive de la température de 5°C.

Dans un mode de réalisation, l'étape de séchage par dessication est réalisée par montée progressive de la température par palier de 1 à 10°C.

Dans un mode de réalisation, l'étape de séchage par dessication est réalisée par montée progressive de la température par palier de 2 à 8°C.

Dans un mode de réalisation, l'étape de séchage par dessication est réalisée par montée progressive de la température par palier de 3 à 7°C.

Dans un mode de réalisation, l'étape de séchage par dessication est réalisée par montée progressive de la température par palier de 5°C.

Dans un mode de réalisation, l'étape de séchage par dessication est réalisée par montée progressive de la température de 5°C.

Dans un mode de réalisation, l'étape de séchage par dessication est réalisée pendant une durée comprise dans un intervalle allant de 1 heure à 12 heures.

Dans un mode de réalisation, l'étape de séchage par dessication est réalisée pendant une durée comprise dans un intervalle allant de 2 heures à 10 heures.

Dans un mode de réalisation, l'étape de séchage par dessication est réalisée pendant une durée comprise dans un intervalle allant de 3 heures à 7 heures.

Dans un mode de réalisation, l'étape de séchage par dessication est réalisée pendant une durée comprise dans un intervalle allant de 4 heures à 6 heures.

Dans un mode de réalisation, l'étape de séchage par dessication est réalisée sous des pressions comprises dans un intervalle allant de 50 à 900 microbars (50 microbars ≤ pressions ≤ 900 microbars).

Dans un mode de réalisation, l'étape de séchage par dessication est réalisée sous des pressions comprises dans un intervalle allant de 100 à 800 microbars (100 microbars ≤ pressions ≤ 800 microbars).

Dans un mode de réalisation, l'étape de séchage par dessication est réalisée sous des pressions comprises dans un intervalle allant de400 à 600 microbars (400 microbars ≤ pressions ≤ 600 microbars).

Dans un mode de réalisation, l'étape de séchage par dessication est réalisée sous des pressions d'environ 600 microbars.

Dans un mode de réalisation, l'étape de séchage par dessication est réalisée sous des pressions d'environ 400 microbars.

Dans un mode de réalisation, l'étape de séchage par dessication est réalisée par montée progressive de la température jusqu'à 35°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 0°C jusqu'à 50°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 2°C jusqu'à 45°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 4°C jusqu'à 40°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 8°C jusqu'à 35°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 0°C jusqu'à 35°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 0°C à 35°C (0°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température dans un intervalle allant de 4°C à 35°C (4°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température jusqu'à 35°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 0°C jusqu'à 50°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 2°C jusqu'à 45°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 4°C jusqu'à 40°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 8°C jusqu'à 35°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 0°C jusqu'à 35°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 0°C à 35°C (0°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température dans un intervalle allant de 4°C à 35°C (4°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température jusqu'à 35°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 0°C jusqu'à 50°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 2°C jusqu'à 45°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 4°C jusqu'à 40°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 8°C jusqu'à 35°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 0°C jusqu'à 35°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 0°C à 35°C (0°C ≤ température ≤ 35°C) et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température dans un intervalle allant de 4°C à 35°C (4°C ≤ température ≤ 35°C) et sous des pressions comprises dans un intervalle allant de 400 à 600 micro-bars (400 micro-bars ≤ pressions ≤ 600 micro-bars).

Dans un mode de réalisation, la ou les couche(s) membranaire(s) issue(s) de tissus placentaires après l'étape a) et/ou contenues dans les éléments de type filaire obtenus à l'étape c) et/ou contenues dans la structure tressée après l'étape e) sur lesquelles on applique l'étape de séchage par dessication présentent un taux d'humidité avant ladite étape de séchage par dessication d'au moins 50 %.

Dans un mode de réalisation, la ou les couche(s) membranaire(s) issue(s) de tissus placentaires après l'étape a) et/ou contenues dans les éléments de type filaire obtenus à l'étape c) et/ou contenues dans la structure tressée après l'étape e) sur lesquelles on applique l'étape de séchage par dessication présente un taux d'humidité avant ladite étape de séchage par dessication d'au moins 60 %.

Dans un mode de réalisation, la ou les couche(s) membranaire(s) issue(s) de tissus placentaires après l'étape a) et/ou contenues dans les éléments de type filaire obtenus à l'étape c) et/ou contenues dans la structure tressée après l'étape e) sur lesquelles on applique l'étape de séchage par dessication présente un taux d'humidité avant ladite étape de séchage par dessication d'au moins 70 %.

Dans un mode de réalisation, la ou les couche(s) membranaire(s) issue(s) de tissus placentaires après l'étape a) et/ou contenues dans les éléments de type filaire obtenus à l'étape c) et/ou contenues dans la structure tressée après l'étape e) sur lesquelles on applique l'étape de séchage par dessication présente un taux d'humidité avant ladite étape de séchage par dessication d'au moins 80 %.

Dans un mode de réalisation, la ou les couche(s) membranaire(s) issue(s) de tissus placentaires après l'étape a) et/ou contenues dans les éléments de type filaire obtenus à l'étape c) et/ou contenues dans la structure tressée après l'étape e) sur lesquelles on applique l'étape de séchage par dessication présente un taux d'humidité avant ladite étape de séchage par dessication d'au moins 90 %.

L'étape de découpe de la ou des couche(s) membranaires issue(s) de tissus placentaires peut être réalisée par tout moyen connue de l'Homme du métier.

Dans un mode de réalisation, l'étape de découpe est réalisée à l'aide d'un scalpel, d'un ciseau, d'une roulette de découpe, d'un appareil de découpe automatisé et/ou d'un emporte-pièce.

L'étape de découpe est réalisée de telle sorte à ce qu'elle permette d'obtenir une structure de type flaire constituées d'au moins une couche membranaire issue de tissus placentaires.

Au sens de la présente invention, on entend par « élément de type filaire » tout objet ayant l'apparence d'un fil et/ou d'une bande et/ou d'un ruban.

Dans un mode de réalisation, les éléments de type filaire ont une largeur et/ou un diamètre compris dans un intervalle allant de 10 $\mu$m à 10 cm (10 $\mu$m $\leq$ largeur et/ou diamètre $\leq$ 10 cm).

Dans un mode de réalisation, les éléments de type filaire ont une largeur et/ou un diamètre compris dans un intervalle allant de 100 $\mu$m à 1 cm (100 $\mu$m $\leq$ largeur et/ou diamètre $\leq$ 1 cm).

Dans un mode de réalisation, les éléments de type filaire ont une largeur et/ou un diamètre compris dans un intervalle allant de 0,5 mm à 5 mm (0,5 mm $\leq$ largeur et/ou diamètre $\leq$ 5 mm).

Dans un mode de réalisation, les éléments de type filaire ont une longueur comprise dans un intervalle allant de 0,5 mm à 10 m (0,5 mm $\leq$ longueur $\leq$ 10 mm).

Dans un mode de réalisation, les éléments de type filaire ont une longueur comprise dans un intervalle allant de 5 cm à 1 m (5 cm $\leq$ longueur $\leq$ 1 m).

Dans un mode de réalisation, les éléments de type filaire ont une longueur comprise dans un intervalle allant de 10 cm à 50 cm (10 cm $\leq$ longueur $\leq$ 50 cm.

On entend par « tressage » ou « structure tressée » l'entrelacement ou le tissage au moins deux éléments de type filaire.

La réalisation du tressage pourra être choisi parmi l'ensemble des motifs connu pour le tissage, le tricotage, le nouage, le tressage, la couture, le crochetage et/ou un nouage à la manière d'un scoubidou.

Dans un mode de réalisation, le tressage est réalisé avec au moins un élément de type filaire constitué d'au moins une couche membranaire issue de tissus placentaires selon l'invention et au moins un élément de type filaire non obtenu par le procédé selon l'invention.

Au sens de la présente invention, on entend par « élément de type filaire non obtenu par le procédé selon l'invention » tout élément de type filaire obtenu par un procédé différent de celui selon la présente invention et/ou à partir d'un matériau différent d'une couche membranaire issue de tissus placentaires.

Les éléments de type filaire non obtenus par le procédé selon l'invention présentent l'avantage de permettre des montages plus complexes et/ou d'apporter des propriétés structurelles et/ou de résistances et/ou de souplesses différentes.

Dans un mode de réalisation, les éléments de type filaire non obtenus par le procédé selon l'invention sont constitués de matériaux naturels et/ou artificiels.

Dans un mode de réalisation, au moins un élément de type filaire non obtenu par le procédé selon l'invention est constitué d'un matériau biocompatible.

Dans un mode de réalisation, au moins un élément de type filaire non obtenu par le procédé selon l'invention est un fil de suture.

Dans un mode de réalisation, le fil de suture est choisi dans le groupe des fils en nylon, en polybutester, en polypropylène et/ou en soie.

Dans un mode de réalisation, le fil de suture est un fil de suture résorbable.

Dans un mode de réalisation, le fil de suture de résorbable est choisi dans le groupe des fils en glycolide et lactide, en polyglytone, en poliglecarpone, en polyglactin, en lactomer, en acide polyglycolique, en glycomer, en polyglyconate ou en polydioxanone.

L'utilisation d'un fil de suture présente l'avantage de disposer d'un matériau biocompatible et pouvant être retiré facilement de la structure tissée avant l'application sur le patient, sans endommager ladite structure. L'utilisation d'un fil de suture résorbable permet d'utiliser directement la structure tissée sans avoir à le retirer de la structure annulaire sans risque pour le patient.

Dans un mode de réalisation, l'un quelconque des procédés selon l'invention comprend le retrait des éléments de type filaire non obtenus par le procédé selon l'invention postérieurement à l'étape d'obtention d'une structure tressée comprenant des éléments de type filaire constitués d'au moins une couche membranaire issue de tissus placentaires.

Dans un mode de réalisation, l'intégralité des éléments de type filaire mis en oeuvre dans le tressage est constituée d'une ou plusieurs couche(s) membranaire(s) issue(s) de tissus placentaires selon l'invention.

Dans un mode de réalisation, le tressage est réalisé au moyen d'un métier à tisser.

Dans un mode de réalisation, le tressage est réalisé avec au moins deux éléments de type filaire.

Dans un mode de réalisation, le tressage est réalisé avec au moins quatre éléments de type filaire.

La présente invention concerne également une structure tressée comprenant des éléments de type filaire constitués d'au moins une couche membranaire issue de tissus placentaires, ladite structure tressée ayant un taux d'humidité supérieur ou égal à 13%.

Dans un mode de réalisation, ladite structure tressée présente un taux d'humidité supérieur ou égal à 20%.

Dans un mode de réalisation, ladite structure tressée présente un taux d'humidité supérieur ou égal à 23%.

Dans un mode de réalisation, ladite structure tressée présente un taux d'humidité compris dans un intervalle allant de 13 à 40% (13% ≤ taux d'humidité ≤ 40 %).

Dans un mode de réalisation, ladite structure tressée présente un taux d'humidité compris dans un intervalle allant de15 a 35% (15% ≤ taux d'humidité ≤ 35 %).

Dans un mode de réalisation, ladite structure tressée présente un taux d'humidité compris dans un intervalle allant de 20 à 27% (20% ≤ taux d'humidité ≤ 27 %).

Au sens de la présente invention, le taux d'humidité est considéré avant toute réhydratation de la structure tressée et/ou avant toute implantation chez un sujet.

Dans un mode de réalisation, ladite structure tressée présente une épaisseur comprise dans un intervalle allant de 0,1mm à 5 mm (0,1 mm ≤ épaisseur ≤ 5 mm).

Dans un mode de réalisation, ladite structure tressée présente une épaisseur comprise dans un intervalle allant de 0,5 mm à 3 mm (0,5 mm ≤ épaisseur ≤ 3 mm).

Dans un mode de réalisation, ladite structure tressée présente une épaisseur comprise dans un intervalle allant de 1 mm à 2 mm (1 mm ≤ épaisseur ≤ 2 mm).

Dans un mode de réalisation, la structure tressée comprenant des éléments de type filaire constitués d'au moins une couche membranaire issue de tissus placentaires selon l'invention est obtenue par un procédé comprenant au moins étape de séchage par dessication.

Dans un mode de réalisation, la structure tressée comprenant des éléments de type filaire constitués d'au moins une couche membranaire issue de tissus placentaires selon l'invention n'est pas obtenue par séchage à l'air libre.

Dans un mode de réalisation, la structure tressée comprenant des éléments de type filaire constitués d'au moins une couche membranaire issue de tissus placentaires selon l'invention n'est pas obtenue par séchage par lyophilisation.

Dans un mode de réalisation, la structure tressée comprenant des éléments de type filaire constitués d'au moins une couche membranaire issue de tissus placentaires selon l'invention comprend uniquement des éléments de type filaire constitués d'une ou plusieurs couche(s) membranaire(s) issue(s) de tissus placentaires.

Dans un mode de réalisation, la structure tressée selon la présente invention est d'origine animale ou humaine.

Dans un mode de réalisation, la structure tressée selon la présente invention est d'origine humaine.

Dans un mode de réalisation, les éléments de type filaire de la structure tressée selon la présente invention comprennent tout ou partie de la membrane amniotique et/ou de la membrane choriale.

Dans un mode de réalisation, les éléments de type filaire de la structure tressée selon la présente invention sont constitués de membrane amniotique humaine.

Dans un mode de réalisation préférentiel, les éléments de type filaire de la structure tressée selon la présente invention comprennent de la membrane amniotique comprenant sa couche spongieuse.

Dans un mode de réalisation, les éléments de type filaire de la structure tressée selon la présente invention comprennent sont constitués de membrane amniotique comprenant sa couche spongieuse.

La présente invention concerne également un dispositif implantable comprenant au moins une structure tressée selon l'invention.

La présente invention concerne également un dispositif implantable comprenant au moins une structure tressée obtenue par l'un quelconque des procédés selon l'invention.

Dans un mode de réalisation, le dispositif implantable est une bandelette sous-urétrale.

La présente invention concerne également une méthode thérapeutique ou chirurgicale de renfort du sphincter urinaire, de renfort du plancher pelvien, de traitement des ulcères de membrane amniotique et/ou des prolapsus, de réparation de tendon et/ou de ligaments, de remplacement ligamentaire, de réparation nerveuse par manchonnage, de réparation cartilagineuse, de remplacement de la dure-mère, de remplacement de membrane sous-muqueuse, de renforcement de la coiffe rotateur, de la membrane amniotiques, des ulcères et/ou des plaies cutanées, et/ou d'amélioration des cicatrisations et/ou des sutures comprenant une étape d'implantation chez un sujet d'une structure tressée comprenant des éléments de type filaire constitués d'au moins une couche membranaire issue de tissus placentaires selon l'invention.

La présente invention concerne également une méthode thérapeutique ou chirurgicale de renfort du sphincter urinaire et/ou du plancher pelvien, de traitement des ulcères de membrane amniotique des hernies et/ou des prolapsus, de réparation de tendon et/ou de ligaments, de remplacement ligamentaire, de réparation nerveuse par manchonnage, de réparation cartilagineuse, de remplacement de la dure-mère, de remplacement de membrane sous-muqueuse, de renforcement de la coiffe rotateur, de la membrane amniotiques, des ulcères et/ou des plaies cutanées, et/ou d'amélioration des cicatrisations et/ou des sutures comprenant une étape d'implantation chez un sujet d'une structure tressée obtenue par l'un quelconque des procédés selon l'invention.

La présente invention concerne également une méthode thérapeutique ou chirurgicale de renfort sphincter urinaire et/ou du plancher pelvien, de traitement des ulcères de membrane amniotique, des hernies et/ou des prolapsus, de réparation de tendon et/ou de ligaments, de remplacement ligamentaire, de réparation nerveuse par manchonnage, de réparation cartilagineuse, de remplacement de la dure-mère, de remplacement de membrane sous-muqueuse, de renforcement de la coiffe rotateur, de la membrane amniotiques, des ulcères et/ou des plaies cutanées, et/ou d'amélioration des cicatrisations et/ou des sutures comprenant une étape d'implantation chez un sujet d'un dispositif implantable selon l'invention.

Dans un mode de réalisation selon l'une quelconque des méthodes thérapeutiques ou chirurgicales selon la présente invention, le sujet est humain ou animal.

Dans un mode de réalisation, la structure tressée comprenant des éléments de type filaire constitués d'au moins une couche membranaire issue de tissus placentaires selon l'invention est utilisable comme matériau d'allogreffe choisi dans le groupe consistant en la greffe de ligament, de fascia lata, de tendon, de dure-mère, de membrane sous-muqueuse, de manchon nerveux, de ligaments, de cartilage, de coiffe rotatrice, et/ou comme agents de traitement du prolapsus, des hernies, de plaies, d'ulcères, de la membrane amniotiques, et/ou des plaies cutanées, et/ou comme agent d'amélioration de sutures et/ ou de la cicatrisation, et/ou pour réparation d'ulcères de membrane amniotique, pour la réparation des tendons, des cartilages et/ou des ligaments et/ou pour la réparation nerveuse par manchonnage, et/ou pour le remplacement des ligaments, de la dure-mère, de membranes sous-muqueuse, et/ou pour le renforcement de la coiffe rotateur, du sphincter urinaire et/ou du plancher pelvien.

Dans un mode de réalisation, la structure tressée comprenant des éléments de type filaire constitués d'au moins une couche membranaire issue de tissus placentaires selon l'invention est utilisable pour le renforcement du sphincter urinaire.

Dans un mode de réalisation, la structure tressée comprenant des éléments de type filaire constitués d'au moins une couche membranaire issue de tissus placentaires selon l'invention est utilisable pour le renforcement du sphincter urinaire dans les pathologies d'incontinence neurologique.

Dans un mode de réalisation, la structure tressée comprenant des éléments de type filaire constitués d'au moins une couche membranaire issue de tissus placentaires selon l'invention est utilisable pour le renforcement du plancher pelvien.

Dans un mode de réalisation, la structure tressée comprenant des éléments de type filaire constitués d'au moins une couche membranaire issue de tissus placentaires selon l'invention est utilisable pour le renforcement du plancher pelvien par voie haute et/ou par voie basse.

Dans un mode de réalisation, la structure tressée comprenant des éléments de type filaire constitués d'au moins une couche membranaire issue de tissus placentaires selon l'invention est utilisable pour le renforcement du plancher pelvien dans le traitement des prolapsus et/ou des hernies des organes pelviens.

Dans un mode de réalisation, la voie basse est la voie trans-vaginale.

Dans un mode de réalisation, la voie haute est la voie péritonéale et/ou trans-abdominale et/ou est réalisée par coelioscopie.

Dans un mode de réalisation, les organes pelviens comprennent la vessie, l'utérus et/ou le rectum.

Dans un mode de réalisation, la structure tressée obtenue par l'un quelconque des procédés selon l'invention est utilisable comme matériau d'allogreffe choisi dans le groupe consistant en la greffe de ligament, de fascia lata, de tendon, de dure-mère, de membrane sous-muqueuse, de manchon nerveux, de ligaments, de cartilage, de coiffe rotatrice, et/ou comme agents de traitement du prolapsus, des hernies, de plaies, d'ulcères, de la membrane amniotiques, et/ou des plaies cutanées, et/ou comme agent d'amélioration de sutures et/ ou de la cicatrisation, et/ou pour réparation d'ulcères de membrane amniotique, pour la réparation des tendons, des cartilages et/ou des ligaments et/ou pour la réparation nerveuse par manchonnage, et/ou pour le remplacement des ligaments, de la dure-mère, de membranes sous-muqueuse, et/ou pour le renforcement de la coiffe rotateur, du sphincter urinaire et/ou du plancher pelvien.

Dans un mode de réalisation, la structure tressée obtenue par l'un quelconque des procédés selon l'invention est utilisable pour le renforcement du sphincter urinaire.

Dans un mode de réalisation, la structure tressée obtenue par l'un quelconque des procédés selon l'invention est utilisable pour le renforcement du sphincter urinaire dans les pathologies d'incontinence neurologique.

Dans un mode de réalisation, la structure tressée obtenue par l'un quelconque des procédés selon l'invention est utilisable pour le renforcement du plancher pelvien.

Dans un mode de réalisation, la structure tressée obtenue par l'un quelconque des procédés selon l'invention est utilisable pour le renforcement du plancher pelvien par voie haute et/ou par voie basse.

Dans un mode de réalisation, la structure tressée obtenue par l'un quelconque des procédés selon l'invention est utilisable pour le renforcement du plancher pelvien dans le traitement des prolapsus et/ou des hernies des organes pelviens.

Dans un mode de réalisation, le dispositif implantable selon l'invention est utilisable comme matériau d'allogreffe choisi dans le groupe consistant en la greffe de ligament, de fascia lata, de tendon, de dure-mère, de membrane sous-muqueuse, de manchon nerveux, de ligaments, de cartilage, de coiffe rotatrice, et/ou comme agents de traitement du prolapsus, des hernies, de plaies, d'ulcères, de la membrane amniotiques, et/ou des plaies cutanées, et/ou comme agent d'amélioration de sutures et/ ou de la cicatrisation, et/ou pour réparation d'ulcères de membrane amniotique, pour la réparation des tendons, des cartilages et/ou des ligaments et/ou pour la réparation nerveuse par manchonnage, et/ou pour le remplacement des ligaments, de la dure-mère, de membranes sous-muqueuse, et/ou pour le renforcement de la coiffe rotateur, du sphincter urinaire et/ou du plancher pelvien.

Dans un mode de réalisation, le dispositif implantable selon l'invention est utilisable pour le renforcement du sphincter urinaire.

Dans un mode de réalisation, le dispositif implantable selon l'invention est utilisable pour le renforcement du sphincter urinaire dans les pathologies d'incontinence neurologique.

Dans un mode de réalisation, le dispositif implantable selon l'invention est utilisable pour le renforcement du plancher pelvien.

Dans un mode de réalisation, le dispositif implantable selon l'invention est utilisable pour le renforcement du plancher pelvien par voie haute et/ou par voie basse.

Dans un mode de réalisation, le dispositif implantable selon l'invention est utilisable pour le renforcement du plancher pelvien dans le traitement des prolapsus et/ou des hernies des organes pelviens.

La présente invention concerne également une structure tressée obtenue par l'un quelconque des procédés selon l'invention pour son utilisation dans le traitement de l'incontinence urinaire, d'un prolapsus et/ou d'une hernie, d'une déchirure et/ou d'une rupture d'un ligament, d'un muscle et/ou d'un tendon.

La présente invention concerne également un dispositif implantable selon l'invention pour son utilisation dans le traitement de l'incontinence urinaire, d'un prolapsus et/ou d'une hernie, d'une déchirure et/ou d'une rupture d'un ligament, d'un muscle et/ou d'un tendon.

La présente invention concerne également une structure tressée comprenant des éléments de type filaire constitués d'au moins une couche membranaire issue de tissus placentaires selon l'invention pour son utilisation dans le traitement de l'incontinence urinaire, d'un prolapsus et/ou d'une hernie, d'une déchirure et/ou d'une rupture d'un ligament, d'un muscle et/ou d'un tendon.

Dans les exemples, tous les pourcentages sont donnés en poids sauf indication contraire, la température est exprimée en degré Celsius sauf indication contraire, les essais sont réalisés à température ambiante sauf indication contraire, et la pression est la pression atmosphérique sauf indication contraire.

## EXEMPLES

**Exemple Ia** : **Exemple d'un mode de réalisation selon l'invention de préparation d'une structure tressée comprenant des éléments de type filaire constitués d'au moins une couche membranaire issue de tissus placentaires avant subi une étape de séchage par dessication.**

Un donneur proprement informé et consentant en accord avec les exigences de la Déclaration d'Helsinki offre en don le tissu placentaire issu d'un accouchement.

Le tissu placentaire est récupéré en salle d'accouchement. Il est congelé à une température de -20°C.

Au laboratoire, en salle stérile, la procédure suivante est appliquée :

En salle stérile, la membrane amniotique est isolée et est nettoyée.

La membrane amniotique subit une succession de bains assurant la viro inactivation de celle-ci. Une agitation douce à environ 30 rpm du milieu liquide est appliquée lors de chaque bain

Dans un premier temps, la membrane amniotique est déposée dans un bain d'eau purifiée à température ambiante pendant environ 3 heures.

Puis, la membrane amniotique est transférée dans un bain décontaminant composé d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la membrane amniotique est transférée dans un bain composé de peroxyde d'hydrogène à 30% w/v à température ambiante pendant environ 15 minutes.

Puis la membrane amniotique est transférée dans un bain décontaminant composé de peroxyde d'hydrogène à 3% w/v à température ambiante pendant environ 1 heure.

L'action chimique appliquée à la membrane amniotique est neutralisée dans deux bains comportant de l'hydroxyde de sodium dilué autour d'un pH de 8,5. Les bains de neutralisation s'effectuent à température ambiante pendant environ 15 minutes.

Afin d'assurer un rééquilibrage du pH et pour éliminer au mieux les résidus organiques se détachant du tissu d'intérêt, la membrane amniotique est transférée dans deux bains successifs de tampon PBS à température ambiante pendant environ 15 minutes afin d'assurer son rééquilibrage physiologique.

Enfin, la membrane amniotique est transférée dans un dernier bain à l'eau purifiée, à température ambiante, pendant 30 minutes.

A cette étape du procédé, la membrane amniotique obtenue selon ce traitement chimique est désinfectée et viro-inactivée.

La membrane amniotique obtenue est découpée à l'aide d'un scalpel. Les éléments de types filaires constituant la chaine (brins déposés dans le sens de la longueur dans le métier à tisser) sont découpés en bandes d'environ 3 à 4 mm de large et environ 30 cm de long. Les éléments de type filaire constituant la trame (ou brin de remplissages, disposés perpendiculairement dans le métier à tisser) sont découpés de la même façon en bandes de 2 à 3 mm de large sans être raccourcis dans le sens de la longueur.

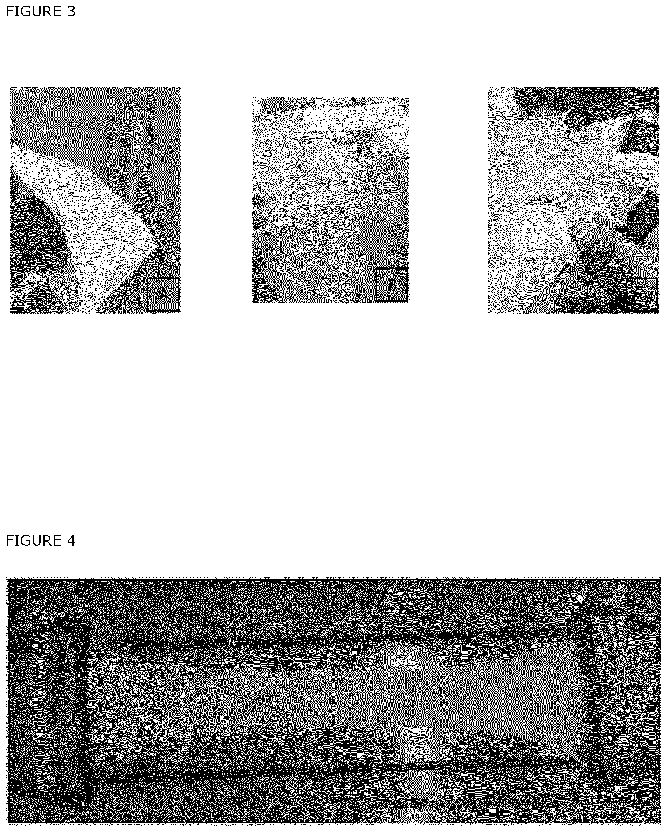

Les éléments de type filaire constitués d'au moins une couche membranaire issue de tissus placentaires ainsi obtenus sont ensuite soumis à une étape de tressage selon la méthode classique de tressage sur un métier à tisser et tel que présenté sur la Figure 4.

On procède ensuite à une étape de séchage par dessication de la structure tressée obtenue. Celle-ci est disposée sur un support en inox au sein d'un lyophilisateur (de type SERAIL CS25-08) et subit une dessication selon le cycle suivant :

a) Température de 8,0°C pendant 5 minutes,
b) Température de 8,0° pendant 120 minutes,
c) Température de 10,0° pendant 30 minutes sous une pression sous vide de 400 $\mu$bar,
d) Température de congélation : 4,0°C
e) Mise sous vide à une pression de 400 $\mu$bar,
f) Température de 10,0°C pendant 30 minutes,
g) Température de 15,0°C pendant 30 minutes,
h) Température de 20,0°C pendant 30 minutes,
i) Température de dessication : 1,0°C,
j) Température de 25,0°C pendant 30 minutes,
k) Température de 30,0°C pendant 30 minutes,
l) Température de 35,0°C pendant 30 minutes,
m) Fin du cycle par un retour à l'atmosphère normale et à la température ambiante pendant une durée de 5 minutes.

La structure tressée déssiquée obtenue est conditionnée à plat en conditions stériles.

En dehors de la salle stérile, une dernière étape de stérilisation de la structure tressée déssiquée est effectuée par exposition de celle-ci à des rayonnements gamma entre 25 et 32 kGrays.

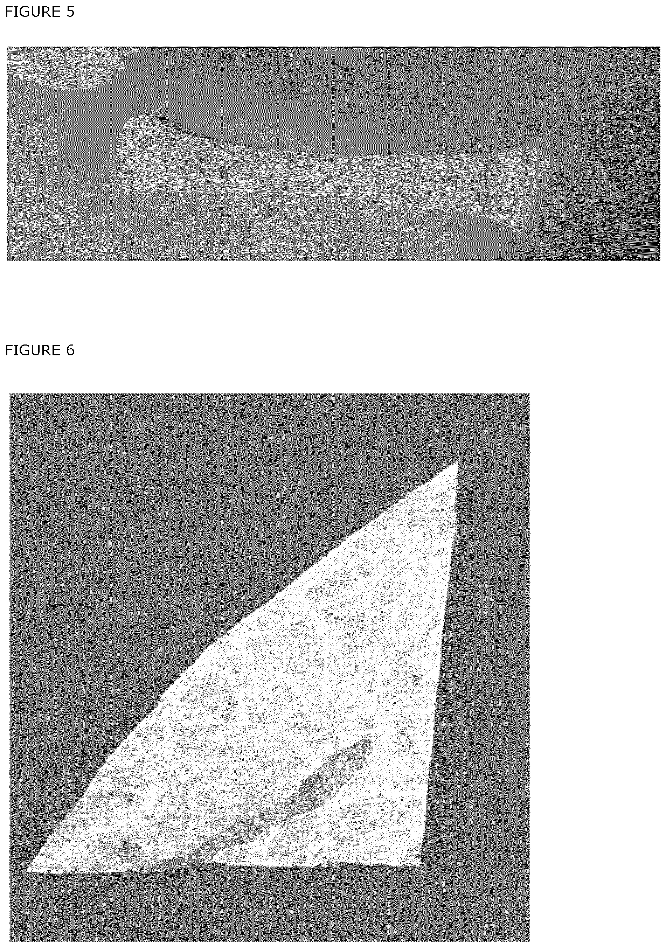

La structure obtenue est photographiée et l'image est présentée en Figure 5.

**Exemple 1a bis: Exemple d'un mode de réalisation selon l'invention de préparation d'une structure tressée comprenant des éléments de type filaire constitués d'au moins une couche membranaire issue de tissus placentaires avant subi une étape de séchage par dessication.**

Un donneur proprement informé et consentant en accord avec les exigences de la Déclaration d'Helsinki offre en don le tissu placentaire issu d'un accouchement.

Le tissu placentaire est récupéré en salle d'accouchement. Il est congelé à une température de -20°C.

Au laboratoire, en salle stérile, la procédure suivante est appliquée :

En salle stérile, la membrane amniotique est isolée et est nettoyée.

La membrane amniotique subit une succession de bains assurant la viro inactivation de celle-ci. Une agitation douce à environ 30 rpm du milieu liquide est appliquée lors de chaque bain

Dans un premier temps, la membrane amniotique est déposée dans un bain d'eau purifiée à température ambiante pendant environ 3 heures.

Puis, la membrane amniotique est transférée dans un bain décontaminant composé d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la membrane amniotique est transférée dans un bain composé de peroxyde d'hydrogène à 30% w/v à température ambiante pendant environ 15 minutes.

Puis la membrane amniotique est transférée dans un bain décontaminant composé de peroxyde d'hydrogène à 3% w/v à température ambiante pendant environ 1 heure.

L'action chimique appliquée à la membrane amniotique est neutralisée dans deux bains comportant de l'hydroxyde de sodium dilué autour d'un pH de 8,5. Les bains de neutralisation s'effectuent à température ambiante pendant environ 15 minutes.

Afin d'assurer un rééquilibrage du pH et pour éliminer au mieux les résidus organiques se détachant du tissu d'intérêt, la membrane amniotique est transférée dans deux bains successifs de tampon PBS à température ambiante pendant environ 15 minutes afin d'assurer son rééquilibrage physiologique.

Enfin, la membrane amniotique est transférée dans un dernier bain à l'eau purifiée, à température ambiante, pendant 30 minutes.

A cette étape du procédé, la membrane amniotique obtenue selon ce traitement chimique est désinfectée et viro-inactivée.

La membrane amniotique obtenue est découpée à l'aide d'un scalpel. Les éléments de types filaires constituant la chaine (brins déposés dans le sens de la longueur dans le métier à tisser) sont découpés en bandes d'environ 3 à 4 mm de large et environ 30 cm de long. Les éléments de type filaire constituant la trame (ou brin de remplissages, disposés perpendiculairement dans le métier à tisser) sont découpés de la même façon en bandes de 2 à 3 mm de large sans être raccourcis dans le sens de la longueur.

Les éléments de type filaire constitués d'au moins une couche membranaire issue de tissus placentaires ainsi obtenus sont ensuite soumis à une étape de tressage selon la méthode classique de tressage sur un métier à tisser et tel que présenté sur la Figure 4.

On procède ensuite à une étape de séchage par dessication de la structure tressée obtenue. Celle-ci est disposée sur un support en inox au sein d'un lyophilisateur (de type SERAIL CS25-08) et subit une dessication selon le cycle suivant :

a) Température de 8,0°C pendant 5 minutes,
b) Température de 8,0° pendant 120 minutes,
c) Température de 10,0° pendant 30 minutes sous une pression sous vide de 400 $\mu$bar,
d) Température de 15,0°C pendant 30 minutes sous une pression sous vide de 400 $\mu$bar,
e) Température de 20,0°C pendant 30 minutes sous une pression sous vide de 400 $\mu$bar,
f) Température de 25,0°C pendant 30 minutes sous une pression sous vide de 200 $\mu$bar,
g) Température de 30,0°C pendant 30 minutes sous une pression sous vide de 200 $\mu$bar,
h) Température de 35,0°C pendant 30 minutes sous une pression sous vide de 200 $\mu$bar,
i) Fin du cycle par un retour à l'atmosphère normale et à la température ambiante pendant une durée de 5 minutes.

La structure tressée déssiquée obtenue est conditionnée à plat en conditions stériles.

En dehors de la salle stérile, une dernière étape de stérilisation de la structure tressée déssiquée est effectuée par exposition de celle-ci à des rayonnements gamma entre 25 et 32 kGrays.

La structure obtenue est photographiée et l'image est présentée en Figure 5.

**Exemple 1b : Exemple comparatif de fabrication d'une structure tressée comprenant des éléments de type filaire constitués d'au moins une couche membranaire issue de tissus placentaires avant subi une étape de séchage à l'air libre.**

Le procédé est réalisé dans les mêmes premières conditions que dans l'exemple 1a) à l'exception de l'étape de séchage par dessication qui est remplacé par un séchage à température ambiante pendant 1 à 2 heures

Les structures obtenues par ce procédé sont photographiée et les images sont présentées en Figure 1 et Figure 2.

**Exemple 2a : Préparation d'une couche membranaire issue de tissus placentaires séchée par dessication.**

Une couche membranaire issue de tissus placentaires est préparée selon le protocole tel que décrit à l'exemple 1a) jusqu'avant l'étape de découpe de la couche membranaire issue de tissus placentaires en éléments de type filaire.

Elle est récupérée et soumise à une étape de séchage par dessication dans les conditions suivantes.

a) Température de 8,0°C pendant 5 minutes,

b) Température de 8,0° pendant 120 minutes,

c) Température de 10,0° pendant 30 minutes sous une pression sous vide de 400 µbar,

d) Température de 15,0°C pendant 30 minutes,

e) Température de 20,0°C pendant 30 minutes,

f) Température de dessication : 1,0°C,

g) Température de 25,0°C pendant 30 minutes,

h) Température de 30,0°C pendant 30 minutes,

i) Température de 35,0°C pendant 30 minutes,

j) Fin du cycle par un retour à l'atmosphère normale et à la température ambiante pendant une durée de 5 minutes.

La structure obtenue est photographiée.

La structure obtenue est présentée sur l'image A de la Figure 3.

**Exemple 2a bis : Préparation d'une couche membranaire issue de tissus placentaires séchée par dessication.**

Une couche membranaire issue de tissus placentaires est préparée selon le protocole tel que décrit à l'exemple 1a) jusqu'avant l'étape de découpe de la couche membranaire issue de tissus placentaires en éléments de type filaire.

Elle est récupérée et soumise à une étape de séchage par dessication dans les conditions suivantes.

k) Température de 8,0°C pendant 5 minutes,

l) Température de 8,0° pendant 120 minutes,

m) Température de 10,0° pendant 30 minutes sous une pression sous vide de 400 µbar,

n) Température de 15,0°C pendant 30 minutes sous une pression sous vide de 400 µbar,

o) Température de 20,0°C pendant 30 minutes sous une pression sous vide de 400 µbar,

p) Température de 25,0°C pendant 30 minutes sous une pression sous vide de 200 µbar,

q) Température de 30,0°C pendant 30 minutes sous une pression sous vide de 200 µbar,

r) Température de 35,0°C pendant 30 minutes sous une pression sous vide de 200 µbar,

s) Fin du cycle par un retour à l'atmosphère normale et à la température ambiante pendant une durée de 5 minutes.

La structure obtenue est photographiée.

La structure obtenue est présentée sur l'image A de la Figure 3.

**Exemple 2b : Exemple comparatif de préparation d'une couche membranaire issue de tissus placentaires séchée par lyophilisation.**

Une couche membranaire issue de tissus placentaires est préparée selon le protocole tel que décrit à l'exemple 1a) jusqu'avant l'étape de découpe de la couche membranaire issue de tissus placentaires en éléments de type filaire.

Elle est récupérée et soumise à un séchage par lyophilisation dans les conditions suivantes.

a) Température de - 10,0°C pendant 5 minutes,

b) Température de - 15,0°C pendant 90 minutes,

c) Température de - 45,0°C pendant 5 minutes,

d) Température de - 45,0°C pendant 90 minutes,

e) Température de 10,0°C pendant 180 minutes sous une pression sous vide de 200 µbar,

f) Température de congélation -40°C,

g) Mise sous vide à une pression de 300 µbar,

h) Température de 10,0°C pendant 210 minutes sous une pression sous vide de 200 µbar,

i) Température de 25,0°C pendant 30 minutes sous une pression sous vide de 200 µbar,

j) Température de 25,0°C pendant 120 minutes sous une pression sous vide de 200 µbar,

k) Température de 35,0°C pendant 60 minutes sous une pression sous vide de 50 µbar,

l) Température de 35,0°C pendant 90 minutes sous une pression sous vide de 50 µbar,

m) Température de 25,0°C pendant 40 minutes sous une pression sous vide de 50 µbar,

n) Température de 25,0°C pendant 20 minutes sous une pression sous vide de 50 µbar,

o) Fin du cycle par un retour à l'atmosphère normale et à la température ambiante pendant une durée de 5 minutes.

La structure obtenue est photographiée. Cette photographie est présentée sur l'image B de la Figure 3.

Exemple 2c : Exemple comparatif de préparation d'une couche membranaire issue de tissus placentaires séchée par séchage à l'air libre.

Une couche membranaire issue de tissus placentaires est préparée selon le protocole tel que décrit à l'exemple 1a) jusqu'avant l'étape de découpe de la couche membranaire issue de tissus placentaires en éléments de type filaire.

La couche membranaire est séchée à température ambiante pendant 1 à 2 heures puis photographiée.

La structure obtenue est présentée sur l'image C de la Figure 3.

**Exemple 3 : Comparaison visuelle entre une couche membranaire issue de tissus placentaires séchée par différentes méthodes.**

En comparant les structures obtenues (voir Figure 3) on observe clairement que la couche membranaire issue de tissus placentaires séchée par au moins une étape de séchage par dessication (image A) est visuellement moins transparente donc plus épaisse et se plie plus naturellement que la couche membranaire séchée par au moins une étape de lyophilisation (image B). La couche membranaire séchée à l'air libre (image C) est la plus rigide et celle qui présente le plus de marques donnant une apparence froissée et la plus transparente donc la plus fine.

**Exemple 4 : Comparaison de la souplesse entre une couche membranaire issue de tissus placentaires séchée par au moins une étape de séchage par dessication et une couche membranaire issue de tissus placentaires séchée par au moins une étape de lyophilisation.**

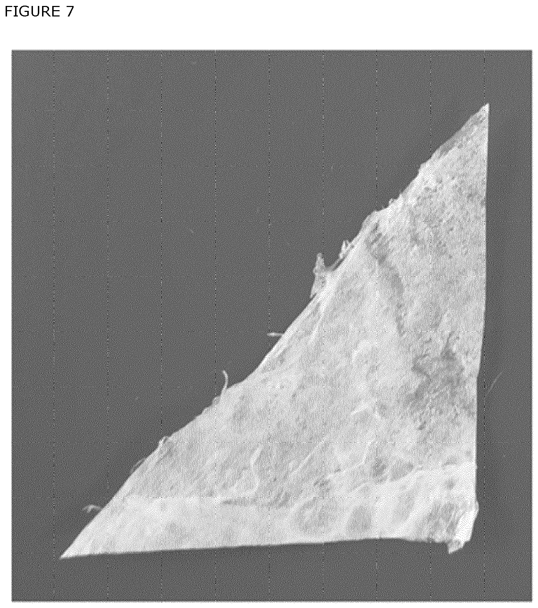

La souplesse est évaluée en pliant chacune des couches membranaires obtenues selon le procédé décrit aux exemples 2a et 2b sur elles-mêmes dans le sens de la diagonale. Les résultats sont présentés en Figure 6 pour la couche membranaire issue de tissus placentaires séchée par au moins une étape de séchage par dessication et en Figure 7 pour la couche membranaire issue de tissus placentaires séchée par au moins une étape de lyophilisation.

On observe clairement que la couche membranaire ayant subi une étape de lyophilisation se replie sur elle-même avec un bord moins arrondi que la couche membranaire dessiquée. En appuyant sur le bord on ressent la possibilité d'une cassure en cas d'appui trop important.

La couche membranaire ayant subi une étape de séchage par dessication est donc plus souple est moins cassante que la membrane amniotique ayant subi une étape de lyophilisation.

**Exemple 5 : Comparaison de l'épaisseur moyenne entre une couche membranaire issue de tissus placentaires séchée par au moins une étape de séchage par dessiccation et une couche membranaire issue de tissus placentaires séchée par au moins une étape de lyophilisation,**

Les couches membranaires obtenues selon le procédé tel que décrit dans l'exemple 2a et 2b sont découpées à l'aide d'un scalpel en éléments de type filaire, plus précisément en bandelettes, d'une largeur d'environ 0,02 mm.

Les bandelettes sont placées verticalement sur une lame et maintenues avec une pince fine en inox dont la taille est préalablement mesurée à l'aide d'un pied à coulisse.

L'ensemble est disposé sous un microscope (Nikon, Eclipse E200) avec un grossissement total de x40 entre l'objectif et l'oculaire. Des photographies sont réalisées pour 4 bandelettes de couches membranaires ayant subi une étape de séchage par dessication selon le protocole tel que décrit dans l'exemple 2a et pour 4 bandelettes de couche membranaire ayant subi une étape de séchage par lyophilisation selon le protocole tel que décrit dans l'exemple 2b.

L'épaisseur des bandelettes et de la pince en inox sont mesurées au pied à coulisse sur les photographies imprimées.

L'épaisseur réelle des bandelettes est calculée à l'aide de la formule suivante :

$$Epaisseur\ réelle\ (mm) = Epaisseur\ sur\ la\ photo\ (mm) \times \frac{Largeur\ réelle\ de\ la\ pince\ (mm)}{Largeur\ de\ la\ pince\ sur\ la\ photo\ (mm)}$$

Des calculs de moyenne et d'écart-type sont réalisés sur un fichier Excel pour déterminer les épaisseurs moyennes des couches membranaires étudiées.

Les résultats sont exposés ci-dessous.

Tableau 1

| | Couche membranaire dessiquée selon l'exemple 2a | Couche membranaire lyophilisée selon l'exemple 2b |
|---|---|---|
| Moyenne | 0,041 | 0,028 |

(suite)

| | Couche membranaire dessiquée selon l'exemple 2a | Couche membranaire lyophilisée selon l'exemple 2b |
|---|---|---|
| Ecart-type | 0,003 | 0,006 |

La membrane amniotique ayant subi une étape de séchage par dessication est donc près de 50% plus épaisse que la membrane amniotique ayant subi une étape de lyophilisation.

**Exemple 6 : Comparaison du taux d'humidité moyen entre une couche membranaire issue de tissus placentaires séchée par différentes méthodes.**

Les paramètres suivants sont rentrés dans un dessiccateur infrarouge (Radwag Mac 50) : Standard / ; 125°C ± 2°C ; programme 5 : variation de poids inférieure à 1 mg/ 120 s.

Une fois le programme démarré, la coupelle est positionnée dans le dessiccateur et tarée.

Environ 50 mg de chaque couche membranaire obtenue selon les exemples 2a, 2b, ou 2c sont déposés dans la coupelle.

La valeur de l'humidité du produit est calculée par le dessiccateur.

Les résultats sont exposés ci-dessous.

Tableau 2

| Échantillons | Couche membranaire dessiquée selon l'exemple 2a | Couche membranaire lyophilisée selon l'exemple 2b | Couche membranaire séchage à l'air selon l'exemple 2c |
|---|---|---|---|
| Humidité (%) | 23,0277 | 12,8874 | < 10,0 |

La couche membranaire dessiquée selon le procédé de l'invention présente une humidité résiduelle près de deux fois supérieure à celle de la couche membranaire ayant subi une étape de lyophilisation et est plus de deux fois supérieur à celle de la couche membranaire séchée à l'air libre.

**Revendications**

1. Procédé de préparation d'une structure tressée comprenant des éléments de type filaire constitués d'au moins une couche membranaire issue de tissus placentaires comprenant les étapes de :

   a) On dispose d'au moins une couche membranaire issue de tissus placentaires,
   b) On découpe la ou les couche(s) membranaire(s) issue(s) de tissus placentaires,
   c) On obtient des éléments de type filaire constitués d'au moins une couche membranaire issue de tissus placentaires,
   d) On tresse les éléments de type filaire,
   e) On obtient une structure tressée comprenant des éléments de type filaire constitués d'au moins une couche membranaire issue de tissus placentaires,

   ledit procédé comprenant au moins une étape de séchage par dessication de la ou des couche(s) membranaire(s) issue(s) de tissus placentaires après l'étape a) et/ou des éléments de type filaire obtenues à l'étape c) et/ou de la structure tressée après l'étape e).

2. Procédé de préparation d'une structure tressée selon la revendication 1, dans lequel l'étape de séchage par dessication est réalisée sur la structure tressée comprenant des éléments de type filaire constitués d'au moins une couche membranaire issue de tissus placentaires obtenue à l'étape e).

3. Procédé de préparation d'une structure tressée selon l'une quelconque des revendications précédentes, dans lequel l'étape de séchage par dessiccation comprend l'application d'une part de la chaleur et d'autre part de vide et/ou d'une matière hygroscopique.

4. Procédé de préparation d'une structure tressée selon l'une quelconque des revendications précédentes, dans lequel dans l'étape de séchage par dessication, la température minimale est choisie de manière à éviter la formation de cristaux d'eau en fonction de la pression appliquée.

5. Procédé de préparation d'une structure tressée selon l'une quelconque des revendications précédente, dans lequel au moins une a couche membranaire issue de tissus placentaires dont on dispose à l'étape a) comprend de la membrane amniotique et/ou de la membrane choriale.

6. Procédé de préparation d'une structure tressée selon l'une quelconque des revendications précédentes, dans lequel au moins une couche membranaire issue de tissus placentaires dont on dispose à l'étape a) comprend la couche spongieuse de la membrane amniotique.

7. Procédé de préparation d'une structure tressée selon l'une quelconque des revendications précédente, ledit procédé comprenant au moins une étape a') de viro-inactivation de la ou des couche(s) membranaire(s) issue(s) de tissus placentaires suite à l'étape a).

8. Procédé de préparation d'une structure tressée selon l'une quelconque des revendications précédente, ledit procédé comprenant en outre une dernière étape de stérilisation de la structure tressée comprenant des éléments de type filaire constitués d'au moins une couche membranaire issue de tissus placentaires obtenue.

9. Structure tressée comprenant des éléments de type filaire constitués d'au moins une couche membranaire issue de tissus placentaires, ladite structure tissée ayant un taux d'humidité supérieur ou égal à 13%.

10. Structure tressée selon la revendication 9, dans laquelle la structure tressée est obtenue par un procédé comprenant au moins étape de séchage par dessication.

11. Structure tressée selon l'une quelconque des revendications 9 ou 10, dans laquelle les éléments de type filaire comprennent de la membrane amniotique et/ou la membrane choriale.

12. Structure tressée selon l'une quelconque des revendications 9 ou 10, dans laquelle les éléments de type filaire comprennent de la membrane amniotique.

13. Structure tressée selon l'une quelconque des revendications 9 à 12, dans laquelle les éléments de type filaire comprennent de la couche spongieuse de la membrane amniotique.

14. Structure tressée selon l'une quelconque des revendications 9 à 13 pour son utilisation dans le traitement de l'incontinence urinaire, d'un prolapsus et/ou d'une hernie, d'une déchirure et/ou d'une rupture d'un ligament, d'un muscle et/ou d'un tendon.

15. Dispositif implantable comprenant au moins une structure tressée obtenue par le procédé selon l'une quelconque des revendications 1 à 8.

16. Dispositif implantable comprenant au moins une structure tressée selon l'une quelconque des revendications 9 à 14.

FIGURE 1

FIGURE 2

FIGURE 3

FIGURE 4

FIGURE 5

FIGURE 6

FIGURE 7

Europäisches
Patentamt

European
Patent Office

Office européen
des brevets

# RAPPORT DE RECHERCHE EUROPEENNE

Numéro de la demande

EP 24 15 5948

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | GRÉMARE AGATHE ET AL: "Development of a vascular substitute produced by weaving yarn made from human amniotic membrane", BIOFABRICATION , vol. 14, no. 4 1 octobre 2022 (2022-10-01), page 045010, XP093107411, UK ISSN: 1758-5082, DOI: 10.1088/1758-5090/ac84ae Extrait de l'Internet: URL:https://iopscience.iop.org/article/10.1088/1758-5090/ac84ae/pdf [extrait le 2023-11-30] * le document en entier * ----- | 1-16 | INV. A61L27/36 A61L27/50 |
| A | US 2008/046095 A1 (DANIEL JOHN [US]) 21 février 2008 (2008-02-21) * revendications 1-20 * ----- | 1-16 | |

**DOMAINES TECHNIQUES RECHERCHES (IPC)**

A61L

Le présent rapport a été établi pour toutes les revendications

| Lieu de la recherche | Date d'achèvement de la recherche | Examinateur |
|---|---|---|
| La Haye | 12 février 2024 | Burtan, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................
& : membre de la même famille, document correspondant

EPO FORM 1503 03.82 (P04C02)

**EP 4 410 323 A1**

## ANNEXE AU RAPPORT DE RECHERCHE EUROPEENNE
## RELATIF A LA DEMANDE DE BREVET EUROPEEN NO.

EP 24 15 5948

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-02-2024

| Document brevet cité au rapport de recherche | Date de publication | Membre(s) de la famille de brevet(s) | Date de publication |
|---|---|---|---|
| US 2008046095 A1 | 21-02-2008 | US 2008046095 A1 | 21-02-2008 |
| | | US 2012294810 A1 | 22-11-2012 |
| | | US 2012294811 A1 | 22-11-2012 |
| | | US 2013006385 A1 | 03-01-2013 |
| | | US 2013317625 A1 | 28-11-2013 |
| | | US 2013337035 A1 | 19-12-2013 |
| | | US 2014283990 A1 | 25-09-2014 |
| | | US 2014330391 A1 | 06-11-2014 |
| | | US 2014370068 A1 | 18-12-2014 |
| | | US 2015110850 A1 | 23-04-2015 |
| | | US 2015174297 A1 | 25-06-2015 |
| | | US 2015182661 A1 | 02-07-2015 |
| | | US 2015209475 A1 | 30-07-2015 |
| | | US 2015265747 A1 | 24-09-2015 |
| | | US 2016030634 A1 | 04-02-2016 |
| | | US 2017157295 A1 | 08-06-2017 |
| | | US 2017319628 A1 | 09-11-2017 |
| | | US 2023201419 A1 | 29-06-2023 |

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2017140914 A
- WO 2021234138 A
- WO 2008061261 A2
- WO 2017140914 A1

**Littérature non-brevet citée dans la description**

- **DEVAL et al.** *J Gynecol Obstet Biol Reprod,* 2002, vol. 31, 131-143
- **GRÉMARE A et al.** *Biofabrication,* 22 Août 2022